# EUROPÄISCHE PATENTANMELDUNG

(11) **EP 4 388 873 A2**
(43) Veröffentlichungstag der Anmeldung: **26.06.2024**
(21) Anmeldenummer: 23217660.2
(22) Anmeldetag: 18.12.2023
(51) Int. Cl.: A01N 25/06, A01N 31/02, A01P 1/00

(54) **HÄNDEDESINFEKTIONSMITTEL ALS ALKOHOLISCHER SCHAUM**

(30) Priorität: 20.12.2022 DE 102022134202
(71) Anmelder: Bode Chemie GmbH, 22525 Hamburg (DE)
(72) Erfinder: Kuboteit, Jana, 21220 Seevetal (DE); Krause-Kyora, Felix, 22119 Hamburg (DE); Eggerstedt, Sven, 22527 Hamburg (DE)
(74) Vertreter: Patentanwälte Olbricht Buchhold Keulertz

(57) **Zusammenfassung**

Gegenstand der Erfindung ist ein schäumbares Desinfektionsmittel enthaltend mindestens einen einwertigen C2 bis C4-Alkohol, mindestens ein Silikontensid und Schaumstabilisatoren, wobei das Desinfektionsmittel als Schaum vorliegt. Die hochalkoholische Lösung des erfindungsgemäßen Desinfektionsmittels ist ein Händedesinfektionsmittel und ist mittels eines Schaumapplikators gut schäumbar. Das Desinfektionsmittel zeigt außerdem eine gute Schaumstabilität trotz des sehr hohen Anteils an Alkohol. Der hohe Alkoholanteil führt erwartungsgemäß zu einer guten antimikrobiellen Wirksamkeit.

## Beschreibung

Gegenstand der Erfindung ist ein schäumendes Desinfektionsmittel, umfassend mindestens einen einwertigen Alkohol mit 2 bis 4 Kohlenstoffatomen, mindestens ein Silikontensid sowie Schaumstabilisatoren. Zudem betrifft die vorliegende Erfindung die Verwendung des Desinfektionsmittels zur Händedesinfektion.

In vielen Bereichen ist eine Händedesinfektion notwendig und teilweise auch gesetzlich vorgeschrieben, wie z. B. in Krankenhäusern, Arztpraxen, in der Lebensmittelindustrie und in der Pharmaindustrie. Aufgrund der guten antibakteriellen Wirksamkeit werden dabei häufig Präparate mit einem hohen Gehalt an einwertigen, niedrigen Alkoholen wie Ethanol, 1-Propanol oder 2-Propanol verwendet. Bei der Händedesinfektion mit alkoholischen Präparaten werden Hände und Unterarme mit dem Desinfektionsmittel eingerieben und für eine halbe bis fünf Minuten feucht gehalten. Auf diese Weise desinfiziert sich beispielsweise Krankenhauspersonal die betroffenen Hautpartien. Es gibt verschiedene Applikationsformen für Händedesinfektionsmittel, wobei neben der klassischen Lösung oder Emulsion, auch Gele oder Schäume eingesetzt werden.

Alkohole besitzen schaumdestabilisierende Eigenschaften. Daher ist es notwendig, in Desinfektionsmittelschäumen zusätzliche Schaumstabilisatoren einzusetzen. Schäumbare alkoholische Händedesinfektionsmittel sind aus dem Stand der Technik bekannt. Die üblicherweise verwendeten Schaumstabilisatoren, wie beispielsweise Cyclomethicon, geraten zunehmend in die Kritik, weil sie von den zuständigen europäischen Behörden, wie der Europäischen Chemikalienagentur (ECHA), als bedenklich eingestuft werden.

EP 3 187 165 A1 beschreibt ein schäumbares Händedesinfektionsmittel, das neben Alkohol und Silikontensiden aromatische Alkohole und zusätzlich Fluortenside als Schaumbildner enthalten. Durch den Zusatz der Fluortenside werden trotz eines hohen Alkoholgehaltes stabile Schäume erreicht.

Aufgabe der vorliegenden Erfindung war es, eine hochalkoholische Zusammensetzung bereitzustellen, die keine als bedenklich eingestuften Schaumstabilisatoren enthält und damit auch strengeren regulatorischen Erfordernissen entsprechen kann. Das hochalkoholische Desinfektionsmittel soll zudem mittels eines Schaumapplikators schäumbar sein und sowohl eine gute Schaumstabilität als auch eine gute antimikrobielle Wirksamkeit aufweisen.

Diese Aufgaben werden mit einem alkoholischen, schäumenden Desinfektionsmittel nach Patentanspruch 1 und einer Verwendung nach Patentansprüchen 12 und 13 gelöst.

Bevorzugte Ausführungsformen der Erfindung mit weiteren Vorteilen sind Gegenstand der Unteransprüche oder nachfolgend beschrieben.

Das erfindungsgemäße alkoholische, schäumende Desinfektionsmittel umfasst
- mindestens einen einwertigen Alkohol mit 2 bis 4 Kohlenstoffatomen,
- mindestens ein Silikontensid,
- mindestens drei Schaumstabilisatoren ausgewählt aus der Gruppe bestehend aus
   i. Citronensäure-Trialkylestern, wobei der Alkylrest ein linearer Alkyrest mit 2 bis 5 Kohlenstoffatomen ist,
   ii. Alkylethercarboxylaten,
   iii. einem Gemisch von Neopentylglycol-Diheptanoat und Isododecan,
   iv. Triheptanoin,
- optional weitere Hilfsstoffe und
- Wasser.

Das erfindungsgemäße alkoholische Desinfektionsmittel ist schäumend, d.h. dass es trotz des Alkoholgehalts bei mechanischer Beanspruchung z.B. in einem Schaumapplikator Schaum bilden kann.

Das Desinfektionsmittel enthält bevorzugt mindestens drei Schaumstabilisatoren, die ausgewählt sind aus der Gruppe bestehend aus
i. Citronensäure-Trialkylester, bevorzugt Triethylcitrat oder Tributylcitrat,
ii. Kaliumlaurylether-4-Carboxylat oder Natriumlaurylether-5-Carboxylat,
iii. einem Gemisch von Neopentylglycol-Diheptanoat und Isododecan, bevorzugt einem Gemisch von 28 bis 32 Gew.-% Neopentylglycol-Diheptanoat und 68 bis 72 Gew.-% Isododecan und
iv. Triheptanoin.

Der Schaumstabilisator der Gruppe i. Citronensäure-Trialkylester ist bevorzugt Triethylcitrat oder Tributylcitrat. Triethylcitrat (CAS-Nr. 77-93-0) wird auch als Citronensäure-Triethylester oder nach IUPAC als 2-Hydroxypropan-1,2,3-tricarbonsäuretriethylester bezeichnet. Tributylcitrat (CAS-Nr. CAS 77-94-1) wird auch als Citronensäuretri-n-butylester oder nach IUPAC als 1,2,3-tributyl-2-hydroxypropan-1,2,3-tricarboxylat bezeichnet.

Der Schaumstabilisator der Gruppe ii. Alkylethercarboxylat ist bevorzugt Kaliumlaurylether-4-Carboxylat (CAS 33939-64-9; IUPAC-Name Poly(oxy-1,2-ethandiyl), alpha-(carboxymethyl)-omega-(dodecyloxy)-Natriumsalz) oder Natriumlaurylether-5-carboxylat (CAS-Nr. 38975-03-0; IUPAC-Name Poly(oxy-1,2-ethandiyl), alpha-dodecyl-omega-carboxymethoxy-Natriumsalz).

Der Schaumstabilisator der Gruppe iii. ist bevorzugt ein Gemisch von 28 bis 32 Gew.-% Neopentylglycol-Diheptanoat und 68 bis 72 Gew.-% Isododecan, wie es beispielweise unter der Bezeichnung LEXFEEL^{®} D4 vertrieben wird.

Der Schaumstabilisator iv. ist Triheptanoin (CAS-Nr. 620-67-7), das nach IUPAC als Propan-1,2,3-triyl trisheptanoat bezeichnet wird.

Die Schaumstabilisatoren sind bevorzugt eine Mischung von Triethylcitrat oder Tributylcitrat mit Kaliumlaurylether-4-Carboxylat oder Natriumlaurylether-5-Carboxylat und mindestens einem dritten Schaumstabilisator aus den Gruppen iii. und iv..

Das Desinfektionsmittel enthält die Schaumstabilisatoren bevorzugt in Mengen von
i. 0,1 bis 1 Gew.-% Citronensäure-Trialkylester,
ii. 0,1 bis 1 Gew.-% Alkalimetalllaurylethercarboxylat,
iii. 0,1 bis 1 Gew.-% Gemisch von Neopentylglycol-Diheptanoat und Isododecan, und/oder
iv. 0,1 bis 1 Gew.-% Triheptanoin,
wenn sie enthalten sind. Die Mengenangaben beziehen sich jeweils auf das Gesamtgewicht des ungeschäumten Desinfektionsmittels.

Das Desinfektionsmittel könnte auch Cocobetain als weiteren bzw. alternativen Schaumstabilisator enthalten. Das Desinfektionsmittel umfasst dann neben dem Alkohol, dem Silikontensid, den optionalen Hilfsstoffen sowie dem Wasser mindestens drei Schaumstabilisatoren ausgewählt aus der Gruppe bestehend aus
i. Citronensäure-Trialkylestern, wobei der Alkylrest ein linearer Alkyrest mit 2 bis 5 Kohlenstoffatomen ist,
ii. Alkylethercarboxylaten,
iii. einem Gemisch von Neopentylglycol-Diheptanoat und Isododecan,
iv. Triheptanoin,
v. Cocobetain.
Cocobetain (CAS-Nr.: 68424-94-2) wird auch als Cocoalkyldimethylbetain oder Cocodimethylglycin bezeichnet und ist z.B. unter dem Handelsnamen Amphosol^{®} DM erhältlich. Wenn der Schaumstabilisator v. enthalten ist, ist er bevorzugt in einer Menge von 0,1 bis 1 Gew.-% enthalten, bezogen auf das Gesamtgewicht des ungeschäumten Desinfektionsmittels. Der Schaumstabilisator v. wird bevorzugt in Mischung mit Triethylcitrat oder Tributylcitrat und mindestens einem dritten Schaumstabilisator aus den Gruppen iii. und iv eigesetzt. Cocobetain stellt vorliegend also insbesondere eine mögliche Alternative zu den Alkylethercarboxylaten dar.

Der einwertige Alkohol mit 2 bis 4 Kohlenstoffatomen ist bevorzugt Ethanol, 1-Propanol, 2-Propanol oder eine Mischung hiervon, bevorzugt Ethanol. Bevorzugt enthält das Desinfektionsmittel 75 Gew.-% bis 90 Gew.-%, bevorzugt 75 - 87 Gew.-%, des einwertigen Alkohols, bezogen auf das Gesamtgewicht des ungeschäumten Desinfektionsmittels. Das erfindungsgemäße Händedesinfektionsmittel enthält außer dem Alkohol bevorzugt keine anderen antimikrobiellen Wirkstoffe.

Bevorzugt enthält das Desinfektionsmittel mindestens ein Silikontensid ausgewählt aus der Gruppe bestehend aus der PEG/PPG-Dimethicone und PEG-Dimethicone. Besonders bevorzugt ist das Silikontensid ausgewählt aus der Gruppe bestehend aus Bis-PEG/PPG-20/20-Dimethicon, PEG/PPG-20/6 Dimethicon, PEG/PPG-4/12 Dimethicon, PEG/PPG-14/4 Dimethicon, PEG-14 Dimethicon, einer Mischung aus Bis-PEG/PPG-20/5 PEG/PPG-20/5 Dimethicon, Methoxy PEG/PPG-25/4 Dimethicon und Caprylic/Capric-Triglyceriden, PEG-17 Dimethicon, PEG/PPG-18/18 Dimethicon, Bis-PEG-18 Methyletherdimethylsilan, PEG/PPG-25/25 Dimethicon, und Cetyl PEG/PPG-10/1 Dimethicon. Insbesondere handelt es sich bei dem Silikontensid um Bis-PEG/PPG-20/20-Dimethicon.

Das mindestens eine Silikontensid ist bevorzugt zu 0,1 Gew.-% bis 5 Gew.-%, bevorzugt 0,5 bis 2 Gew.-%, im Desinfektionsmittel enthalten, bezogen auf das Gesamtgewicht des ungeschäumten Desinfektionsmittels.

In einer Ausführungsform enthält das alkoholische, schäumende Desinfektionsmittel
75 bis 90 Gew.-% mindestens eines einwertigen Alkohols,
0,5 bis 2 Gew.-% mindestens eines Silikontensids,
1,0 bis 2,0 Gew.-% Schaumstabilisatoren ausgewählt aus den Gruppen i., ii., iii und/oder iv.,
0 bis 3 Gew.-%, bevorzugt 0,15 bis 2,5 Gew.-%, weitere Hilfsstoffe und
Wasser,
jeweils bezogen auf das Gesamtgewicht des ungeschäumten Desinfektionsmittels.

Bevorzugt enthält das alkoholische, schäumende Desinfektionsmittel

| | |
|---|---|
| 75 bis 90 Gew.-% | Ethanol, 1-Propanol, 2-Propanol oder eine Mischung hiervon, |
| 0,5 bis 2 Gew.-% | PEG/PPG-Dimethicon und/oder PEG-Dimethicon, |
| 1,0 bis 2,0 Gew.-% | einer Schaumstabilisatormischung bestehend aus Triethylcitrat oder Tributylcitrat, Kaliumlaurylether-4-Carboxylat oder Natriumlaurylether-5-Carboxylat und mindestens einem dritten Schaumstabilisator aus den Gruppen iii. und iv., |
| 0 bis 3 Gew.-% | weitere Hilfsstoffe, bevorzugt Hautbefeuchter, Hautpflegestoffe, Farbstoffe und/oder Parfüm, und |

Wasser,
jeweils bezogen auf das Gesamtgewicht des ungeschäumten Desinfektionsmittels.

In einer Ausführungsform besteht das Desinfektionsmittel aus
75 bis 90 Gew.-% mindestens eines einwertigen Alkohols,
0,5 bis 2 Gew.-% mindestens eines Silikontensids,
1,0 bis 2,0 Gew.-% Schaumstabilisatoren,
0,1 bis 1,0 Gew.-% Glycerin oder Glycerinmischung,
0 bis 2 Gew.-%, bevorzugt 0,1 bis 2 Gew.-%, weitere Hilfsstoffe und
Wasser,
jeweils bezogen auf das Gesamtgewicht des ungeschäumten Desinfektionsmittels, wobei sich die Komponenten zu 100 Gew.-% ergänzen.

Bevorzugt enthält das Desinfektionsmittel als Hautpflegemittel Glycerin oder ein Glycerin-Gemisch. Glycerin oder das Glycerin-Gemisch ist bevorzugt in Konzentrationen von 0,1 bis 1 Gew.-% enthalten, wenn es zugesetzt wird. Der Desinfektionsmittelschaum kann weitere Hautpflegestoffe enthalten, u.a. natürliche Feuchthaltefaktoren und Pflegestoffe wie z.B. Isopropy-Imyristat, Tocopherylacetat, Jojobaölester, 1,3-Propandiol, Heptamethylnonan, Bisabolol, Panthenol, Allantoin. Außerdem können weitere Hilfsstoffe enthalten sein, die in alkoholischen Händedesinfektionsmitteln üblicherweise eingesetzt werden, z.B. Tenside wie Lauryldimethylamin oder Laurindiethanolamid.

Das erfindungsgemäße schäumende, alkoholische Desinfektionsmittel wird zur Händedesinfektion verwendet. Unter Händedesinfektion wird ein Vorgang verstanden, der in kurzer Zeit eine möglichst starke Reduktion der transienten, mikrobiellen Handflora erreicht, so dass eine Übertragung von mikrobieller Kontamination an Dritte durch die Hände vermieden wird. Je nachdem, ob es sich um eine hygienische oder um eine chirurgische Händedesinfektion handelt, beträgt die Einwirkzeit des Händedesinfektionsmittels eine halbe bis eine Minute (geltend für die hygienische Händedesinfektion) bzw. eine bis fünf Minuten (geltend für die chirurgische Händedesinfektion), in denen die Hände feucht gehalten werden. Nach einer erfolgreichen Händedesinfektion können die Hände als hygienisch sicher betrachtet werden. Alkoholische Zusammensetzungen mit einem hohen Alkoholgehalt wie das erfindungsgemäße Händedesinfektionsmittel haben den Vorteil, dass sie bereits nach kurzer Einwirkzeit von nur 0,25 bis 1 Minute eine umfassende antimikrobielle Wirksamkeit in Suspensionsversuchen nach Europäischen Normen, z. B. EN13727, EN13624, EN14476, aufweisen.

Das schäumende Desinfektionsmittel enthält zur Schaumstabilisierung mindestens drei Schaumstabilisatoren. Die als Schaumstabilisatoren eingesetzten Substanzen waren bisher nur als Weichmacher bekannt, nicht aber mit einer schaumstabilisierenden Wirkung beschrieben. Besonders die Verwendung von Alkylethercarboxylaten führt in Kombination mit den Citronensäuretriestern und mindestens einem weiteren neuen Schaumstabilisator zu einer deutlich besseren Konsistenz und Stabilität des alkoholischen Schaums. Alle Substanzen aus den Gruppen i., ii., iii. und iv. tragen zur Schaumstabilisierung bei.

Gleichzeitig ist die Haptik des alkoholischen Schaums, d. h. das Hautgefühl, wichtig. Das Desinfektionsmittel zeigt sowohl ein gutes Schaumverhalten als auch gleichzeitig eine gute Haptik, da es nicht klebt und ein gutes Hautgefühl aufweist.

Zur Verwendung als Händedesinfektionsmittel muss der alkoholische Schaum zunächst fest sein, d. h. er darf beim Applizieren nicht gleich zerfließen und nicht tropfen. Der Schaum darf aber nicht zu fest sein und darf nicht zu lange Schaum bleiben, da er sich auf der Hand verteilen lassen muss. Das bedeutet, dass der Schaum beim Applizieren fest sein muss, sich aber innerhalb von 15-30 Sekunden auflösen und auf der Hand verteilen muss. Bei der Verteilung auf der Hand verschwindet der Schaum.

Die hochalkoholische Lösung des erfindungsgemäßen Desinfektionsmittels ist mittels eines Schaumapplikators, insbesondere eines drucklosen Schaumapplikators, gut schäumbar. Geeignet sind beispielsweise Schaumapplikatoren, die je nach Ausführungsform zwischen 0,75 ml, 1,0 ml, 1,2 ml und 1,5 ml Flüssigkeit pro Hub pumpen, entsprechend einer Schaumdosis von 9 ml, 10 ml oder 12 ml. Beispielsweise werden Schaumapplikatoren verwendet, die 1 ml Flüssigkeit zu ca. 10 ml Schaum aufschäumen.

Das erfindungsgemäße Desinfektionsmittel wird in Form eines alkoholischen Schaums appliziert, der auf einem hochalkoholischen wässrigen System basiert. Die Applikation erfolgt bevorzugt mittels eines drucklosen Schaumapplikators. Der Schaumapplikator ist bevorzugt ein Schaumdispenser oder eine Schaumpumpe.

Ein hoher Alkoholgehalt ist wünschenswert, um mit nur einer kleinen Menge (ca. 3 ml) die notwendige Wirksamkeit zu erreichen. Hier wird auf die Richtlinie EN 1500 "hygienische Händedesinfektion" verwiesen. Bei einem hohen Alkoholgehalt reichen in der Regel zwei bis drei Hübe aus dem Schaumapplikator aus, um die benötigte Menge zu erhalten.

Das erfindungsgemäße Desinfektionsmittel zeigt trotz des typischerweise sehr hohen Anteils an Alkohol eine gute Schaumstabilität. Der hohe Alkoholanteil führt erwartungsgemäß zu einer guten antimikrobiellen Wirksamkeit. Die Wirksamkeit nach EN1500 (hygienische Händedesinfektion) und EN14476 (viruzide Wirksamkeit) ist mit einer Anwendungszeit von 30 s belegt. Das Desinfektionsmittel besitzt also eine gute antimikrobielle Wirksamkeit und bildet einen stabilen hochalkoholischen Schaum. Zudem sind die verwendeten Schaumstabilisatoren umwelt- und hautverträglich.

### Beispiele

Die Erfindung wird anhand der Beispiele näher erläutert.

Die Herstellung der schaumfähigen Lösungen erfolgt in einem einfachen Mischprozess. Im ersten Schritt werden die flüssigen Komponenten Alkohol und Wasser vorgelegt. Die weiteren Inhaltsstoffe werden dann nacheinander unter Rühren zugegeben und so lange gerührt bis eine klare Lösung vorliegt.

Es wurden erfindungsgemäße Desinfektionsmittel gemäß den in Tabelle 1 dargestellten Rezepturen hergestellt.

**Tabelle 1: Rezepturen der hergestellten erfindungsgemäßen Desinfektionsmittel**

| **Beispiel Nr.** | **1** | **2** | **3** | **4** | **5** | **6** | **7** | **8** |
|---|---|---|---|---|---|---|---|---|
| **Komponente** | [%] | [%] | [%] | [%] | [%] | [%] | [%] | [%] |
| **Ethanol 99%, 2-Butanon vergällt** | 85,86 | 85,86 | 85,86 | 80,81 | - | 85,86 | 85,86 | 85,86 |
| **2-Propanol** | - | - | - | - | 45,00 | - | - | - |
| **1-Propanol** | - | - | - | - | 30,00 | - | - | - |
| **Bis-PEG/PPG-20/20 Dimethicon** | 1,00 | 1,00 | 1,00 | 1,00 | 1,00 | 1,00 | 1,00 | 1,00 |
| **Triethylcitrat** | 0,50 | 0,50 | 0,50 | 0,50 | 0,50 | - | 0,50 | - |
| **Tributylcitrat** | - | - | - | - | - | 0,50 | - | 0,50 |
| **Kaliumlaurylether-4 Carboxylat** | 0,40 | 0,50 | 0,98 | 0,40 | 0,40 | 0,40 | - | - |
| **Natriumlaurylether-5 Carboxylat** | - | - | - | - | - | - | 0,40 | 0,40 |
| **LexFeel D4*** | - | 0,40 | - | 0,40 | - | - | - | - |
| **Triheptanoin** | 0,40 | - | 0,40 | - | 0,40 | 0,40 | 0,40 | 0,40 |
| **Isopropylmyristat** | 0,75 | 0,75 | 0,75 | 0,75 | 0,75 | 0,75 | 0,75 | 0,75 |
| **Glycerin 85 %** | 0,50 | - | - | 0,50 | 0,50 | 0,50 | 0,50 | 0,50 |
| **Hydagen Aquaporin*** | - | 0,40 | 0,40 | - | - | - | - | - |
| **Floraester K-20W Jojoba*** | 0,1 | 0,1 | 0,1 | 0,1 | 0,1 | 0,1 | 0,1 | 0,1 |
| **Tocopherolacetat** | 0,1 | 0,1 | 0,1 | 0,1 | 0,1 | 0,1 | 0,1 | 0,1 |
| **gereinigtes Wasser (Aqua purificata)** | 10,39 | 10,39 | 9,91 | 15,44 | 21,25 | 10,39 | 10,39 | 10,39 |

| | | | | | | | | |
|---|---|---|---|---|---|---|---|---|
| *LexFeel^{®} D4: Gemisch aus Neopentylglycol-Diheptanoat und Isododecan *Hydagen Aquaporin^{®}: Gemisch Glycerylglucoside und Glycerin *Floraester K-20W Jojoba^{®}: hydrolysierte Jojobaester mit Wasser | | | | | | | | |

Zum Vergleich wurden nicht erfindungsgemäße Desinfektionsmittel mit nur einem einzigen Schaumstabilisator (V1) oder höchstens zwei Schaumstabilisatoren (V2) hergestellt. Die Rezepturen der Vergleichsbeispiele sind in Tabelle 2 angegeben.

**Tabelle 2: Rezepturen der als Vergleich hergestellten Desinfektionsmittel**

| **Beispiel Nr.** | **V1** | **V2** |
|---|---|---|
| **Komponente** | [%] | [%] |
| **Ethanol 99%, 2-Butanon vergällt** | 85,86 | 85,86 |
| **Bis-PEG/PPG-20/20 Dimethicon** | 1,00 | 1,00 |
| **Triethylcitrat** | 1,00 | 0,50 |
| **Kaliumlaurylether-4 Carboxylat** | - | - |
| **Natriumlaurylether-5 Carboxylat** | - | - |
| **LexFeel^{®} D4** | - | 0,35 |
| **Triheptanoin** | - | - |
| **Isopropylmyristat** | 1,00 | 1,00 |
| **Glycerin 85 %** | 0,50 | 0,50 |
| **Hydaqen Aquaporin^{®}** | - | - |
| **Floraester K-20W Jojoba^{®}** | - | 0,10 |
| **Tocopherolacetat** | - | 0,10 |
| **Lauraminoxid** | - | 0,35 |
| **gereinigtes Wasser (Aqua purificata)** | 10,64 | 10,14 |

Mit den Desinfektionsmitteln gemäß der Vergleichsbeispiele war es nicht möglich einen stabilen Schaum zu erzeugen, der sich auf die Hände applizieren lässt. Desinfektionsmittel V1 mit nur einem einzigen Schaumstabilisator bildete einen instabilen Schaum mit großen Blasen, der beim Pumpen mit dem Schaumapplikator spritzte und nach dem Auftragen so schnell zusammenfiel, dass er von der Hand lief und tropfte. Desinfektionsmittel V2 mit zwei Schaumstabilisatoren bildete einen etwas stabileren Schaum, behielt die Schaumstabilität aber auch nicht ausreichend lange für eine Applikation in gewünschter Qualität bei.

### Schäumbarkeit

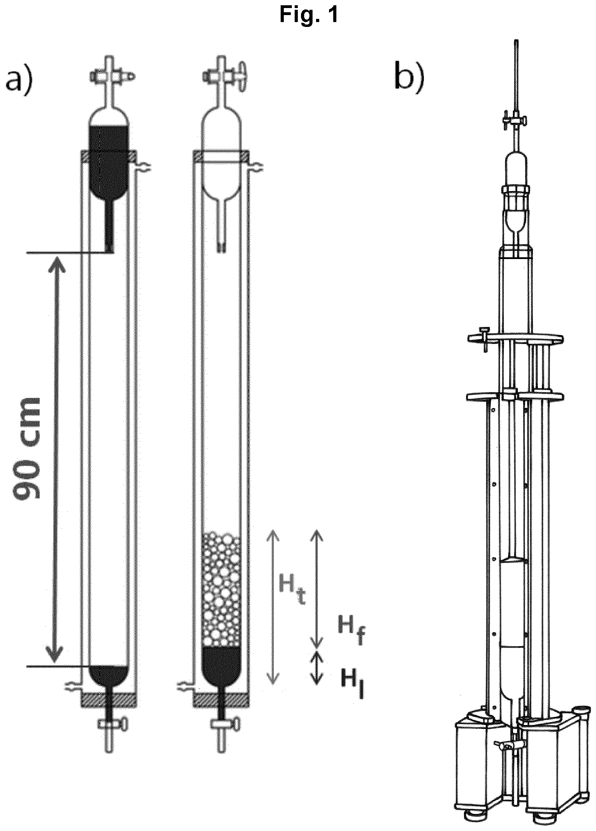

Die Bewertung der Schäumbarkeit der Beispiele 1 bis 8 erfolgte mittels Ross Miles Foam Analyser (RMFA) der Firma Krüss gemäß ASTM D1173-07 bei 20°C (siehe auch Figur 1).

Figur 1a zeigt eine Darstellung einer Ross-Miles-Schaumanalyse vor (links) und nach der Schaumbildung (rechts). Die Pfeile geben die Gesamthöhe Ht, die Flüssigkeitshöhe HI und die Nettoschaumhöhe Hf wie vom Instrument erfasst an. Figur 1 b zeigt eine Darstellung des KRÜSS RMFA für die automatische Schaumanalyse nach Ross-Miles-Norm ASTM D 1173 07.

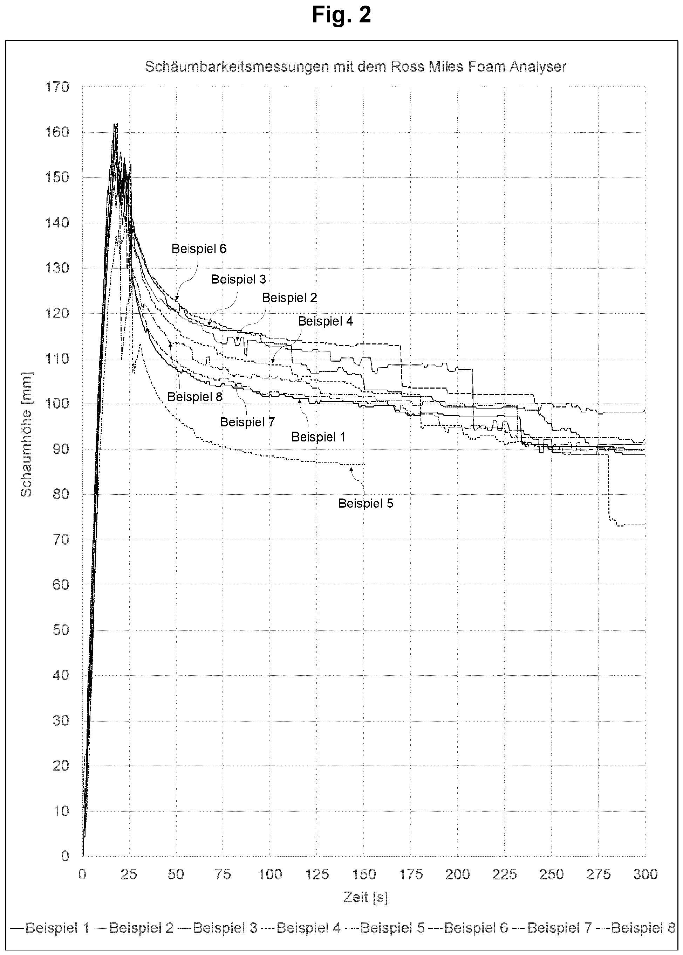

Die Ergebnisse der Schäumbarkeitsmessungen sind in Figur 2 dargestellt. Die Messungen wurden im Fall von Beispiel 5 über einen Zeitraum von 150 Sekunden und bei den anderen Beispielen über einen Zeitraum von 300 Sekunden durchgeführt. Zwischen 0 und 25 Sekunden schäumt sich das Produkt durch sich selbst auf. Dieser Bereich ist vergleichbar mit dem Aufschäumen des Produktes durch eine Schaumpumpe. Der Anwendungszeitraum liegt zwischen 25 und maximal 75 Sekunden.

Es zeigt sich, dass bei den erfindungsgemäßen Händedesinfektionsmitteln eine deutlich größere Schaumhöhe erreicht werden kann, d.h. dass die erfindungsgemäßen Zusammensetzungen besser schäumen. Zudem ist der Schaum bei den erfindungsgemäßen Zusammensetzungen über das Intervall von 0 Sekunden bis 5 Minuten deutlich stabiler als bei den Vergleichsrezepturen.

### Antimikrobielle Wirksamkeit

Das Desinfektionsmittel wurde hinsichtlich seiner antimikrobiellen Wirksamkeit untersucht. Auf Grund des hohen Alkoholgehalts ist eine bakterizide Wirksamkeit nach Methode EN 13727: 2015 ebenso gegeben, wie eine Wirksamkeit gegen Hefen nach Methode EN 13624: 2021.

Es wurden erfindungsgemäße Desinfektionsmittel gemäß der in Tabelle 3 dargestellten Rezepturen hergestellt.

**Tabelle 3: Rezepturen der hergestellten erfindungsgemäßen Desinfektionsmittel**

| **Beispiel Nr.** | **9** | **10** | **11** |
|---|---|---|---|
| **Komponente** | [%] | [%] | [%] |
| **Ethanol 99%, 2-Butanon vergällt** | 85,86 | 85,86 | 85,86 |
| **Kaliumlaurylether-4 Carboxylat** | 0,40 | 0,50 | 0,98 |
| **Triethylcitrat** | 0,50 | 0,50 | 0,50 |
| **LexFeel^{®} D4** | - | 0,40 | - |
| **Triheptanoin** | 0,40 | - | 0,40 |
| **Isopropylmyristat** | 0,75 | 0,75 | 0,75 |
| **Glycerin 85 %** | 0,50 | - | - |
| **Hydaqen Aquaporin^{®}** | - | 0,40 | 0,40 |
| **Bis-PEG/PPG-20/20 Dimethicon** | 1,00 | 1,00 | 1,00 |
| **Floraester K-20W Jojoba^{®}** | 0,10 | 0,10 | 0,10 |
| **Tocopherolacetat** | 0,10 | 0,10 | 0,10 |
| **gereinigtes Wasser (Aqua purificata)** | 10,39 | 10,39 | 9,91 |

Zur Messung der viruziden Wirksamkeit wurde u.a. die Wirksamkeit gegen Adenovirus getestet. Die Überprüfung der Wirksamkeit gegen Adenovirus erfolgte nach der EN14476:2019 unter normaler Belastung (clean conditions). Getestet wurden Desinfektionsmittel gemäß den in Tabelle 3 angegebenen Beispielen 9, 10 und 11. Dabei konnte für das erfindungsgemäße Desinfektionsmittel eine ausreichende Wirksamkeit gegen Adenovirus innerhalb von 15 s belegt werden. Es konnte eine Reduktion von RF ≥ 5,5 in 30 sec erreicht werden. Zudem konnte für das erfindungsgemäße Desinfektionsmittel auch eine sehr schnelle Wirksamkeit gegen Poliovirus nachgewiesen werden. Die Überprüfung der Wirksamkeit gegen Poliovirus erfolgte nach der EN14476:2019 unter hoher Belastung (dirty conditions). Dabei konnte eine Reduktion von RF ≥ 4 in 15 sec erreicht werden. Das Desinfektionsmittel kann somit als "viruzid" ausgelobt werden.

Es wurde die Wirksamkeit bei der hygienischen Händedesinfektion gemäß der Methode EN 1500:2013 getestet. Die Prüfung der Eignung für die hygienische Händedesinfektion erfolgt mit dem Testkeim Escherichia Coli. Es werden 3 ml Probe in den trockenen Händen für 30 Sekunden verrieben. Nach EN 1500 (Stand April 2013, Screening mit 10 Probanden) zeigten die erfindungsgemäßen Desinfektionsmittel die erforderliche Reduktion der Testkeime.

Zudem wurden mit einem erfindungsgemäßen Desinfektionsmittel verschiedene antimikrobielle Wirksamkeiten getestet. Tabelle 4 zeigt eine Übersicht über die erreichten Keimreduktionen.

**Tabelle 4: Antimikrobielle Wirksamkeiten und Testmethoden**

| **Methode** | **Keim** | **Bedingung** | **Prüfkonzentration [%]** | **Reduktionszeit** | **Reduktionsfaktor** |
|---|---|---|---|---|---|
| EN 1500 | Staphylococcus aureus | | | 30 s /3 mL | erfüllt |
| EN 14476:2013 + A2:2019 | Modified Vaccinavirus Ankara (MVA) | clean | 80 | 15s | ≥ 5,79 |
| EN 14476:2013 + A2:2019 | Adenovirus | clean | 80 | 15s | ≥ 5,61 |
| EN 14476:2013 + A2:2019 | Murine Norovirus (MNV) | clean | 80 | 15s | ≥ 5,18 |
| EN 14476:2013 + A2:2019 | Poliovirus | clean | 80 | 15s | ≥ 4,18 |
| EN 13727:2012 +A2:2015 | Staphylococcus aureus | clean | 80 | 15s | ≥ 5 |
| | Enterococcus hirae | clean | 80 | 15s | ≥ 5 |
| | Escherichia coli K12 | clean | 80 | 15s | ≥ 5 |
| | Pseudomonas aeruginosa | clean | 80 | 15s | ≥ 5 |
| EN 13624:2021 | Candida albicans | clean | 80 | 15s | ≥ 4 |
| EN 14348:2005 | Mycobacterium terrae | clean | 80 | 15s | ≥ 4 |
| EN 14348:2005 | Mycobacterium avium | clean | 80 | 15s | ≥ 4 |

Es konnte gezeigt werden, dass das erfindungsgemäße Desinfektionsmittel gegen Viren, Polioviren, Pilze, Mykobakterien und auch Bakterien wirksam ist und innerhalb von 15 sec jeweils die geforderte Keimreduktion erreicht. Daher ist eine Auslobung als bakterizid, viruzid, levurozid, mykobakterizid und tuberkulozid möglich.

### Hautverträglichkeit

Im Rahmen einer Hautstudie wurde ein erfindungsgemäßes Desinfektionsmittel an 20 Probanden für 14 Tage 20-mal täglich angewendet. Die Hautfeuchte wurde mittels Corneometrie und kapazitiver Kontaktbildgebung (Capacitive Contact Imaging - CCI, Epsilon Model E100) gemessen. Zudem erfolgte eine visuelle Beurteilung durch einen Dermatologen vor der ersten sowie nach der letzten Anwendung und eine Befragung der Probanden zu den Produkteigenschaften.

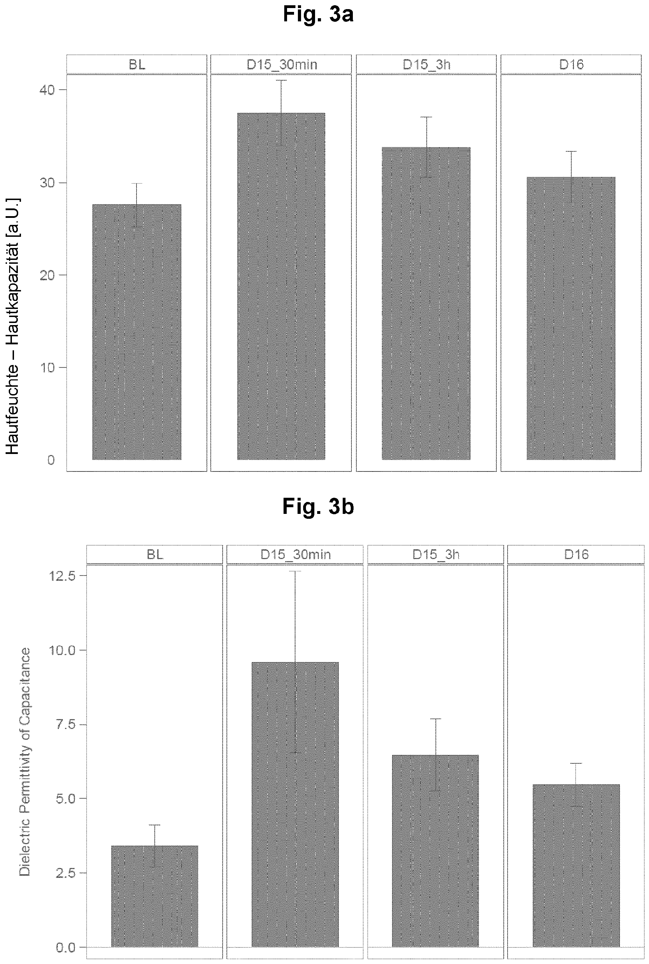

Es konnte eine signifikante Erhöhung der Hautfeuchte mittels Corneometrie und kapazitiver Kontaktbildgebung (CCI) gemessen werden. So zeigt Figur 3a die Mittelwerte der Hautfeuchtigkeit - Hautkapazität in arbiträren Einheiten [arbitrary units (a.u.)] gemessen mittels Corneometer als Balkendiagramm mit Mittelwerten und 95 % Konfidenzintervallen der Rohdaten (n = 19 - 20). D bedeutet Tag (day) und D16 steht für Tag 16 nach Studienbeginn. BL bedeutet Baseline und steht für den Wert der Grundlinie ohne Auftrag eines Mittels auf die Haut.

Es sind folgende Messwerte dargestellt:
BL: Baseline-Bewertung vor der ersten Anwendung des Testprodukts
Tag 15_30min: 30 Minuten nach der letzten Anwendung
Tag 15_3h: 3 Stunden nach der letzten Anwendung
Tag 16: 24 Stunden nach der letzten Anwendung

Figur 3b zeigt die Dielektrizitätskonstante der Kapazität durch kapazitive Kontaktbildgebung (CCI) als Balkendiagramm mit Mittelwerten und 95 % Konfidenzintervallen der Rohdaten (n = 19 - 20).

Die Beurteilung zur Hautverträglichkeit durch den Dermatologen zeigte tendenziell eine Verringerung der Trockenheit, Rötung und Schuppung der Haut. Dies entsprach auch dem Empfinden der Probanden.

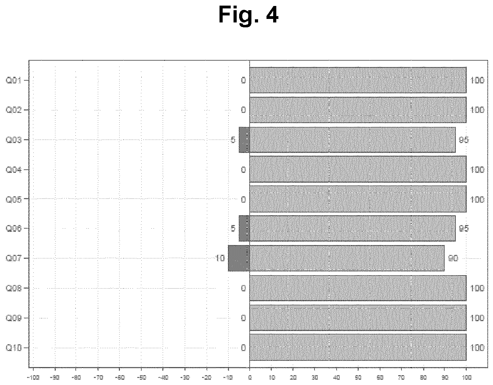

Nach der ersten Anwendung füllten die Probanden einen Fragebogen zur Bewertung des Produktes aus, der eine sehr positive Wahrnehmung des Produktes widerspiegelt. Die Auswertung des Fragebogens ist in Figur 4 dargestellt. Die linken Balken im negativen Wertebereich der x-Achse zeigen Ablehnung an und die rechten Balken im positiven Wertebereich der x-Achse zeigen Zustimmung an (in %). Es wurden folgende Fragen gestellt, wobei die Probanden jeweils "Stimme zu" oder "Stimme nicht zu" angeben konnten.
Frage Q01: Das Testprodukt vermittelt ein angenehmes Hautgefühl
Frage Q02: Das Testprodukt pflegt die Haut schon während der Desinfektion
Frage Q03: Das Testprodukt vermittelt ein glattes Hautgefühl
Frage Q04: Das Testprodukt vermittelt ein weiches / geschmeidiges Hautgefühl
Frage Q05: Das Testprodukt spendet sofortige Feuchtigkeit
Frage Q06: Das Testprodukt ist angenehm zu verteilen
Frage Q07: Das Testprodukt ist nicht klebrig
Frage Q08: Das Testprodukt ist besser als mein normalerweise verwendetes Desinfektionsmittel (nur für Personen, die normalerweise ein Desinfektionsmittel verwenden)
Frage Q09: Ich würde das Testprodukt weiterempfehlen
Frage Q10: Ich würde das Testprodukt kaufen

Es zeigte sich, dass bereits nach einmaliger Anwendung die pflegenden Eigenschaften und allgemeinen Produkteigenschaften des getesteten Handdesinfektionsschaums von den Probanden sehr positiv bewertet wurden.

### Meßmethoden

Corneometrie ist eine Methode zur Bestimmung der Hautfeuchte. Dabei wird mit Hilfe eines Sensors die Dielektrizitätskonstante der obersten Hautschichten bestimmt. Die Dielektrizitätskonstante variiert je nach Wassergehalt der Haut.

Kapazitive Kontaktbildgebung (Capacitive Contact Imaging - CCI, Epsilon Model E100): Bei der kapazitiven Kontaktbildgebung wird mit Hilfe eines Sensors die Dielektrizitätskonstante der obersten Hautschichten bestimmt. Die Dielektrizitätskonstante variiert je nach Wassergehalt der Haut. Die Technologie liefert bildgebende Daten und Daten zur Hautfeuchte gleichzeitig.

Die Erfindung ist nicht auf eine der vorbeschriebenen Ausführungsformen beschränkt, sondern in vielfältiger Weise abwandelbar.

Sämtliche aus den Ansprüchen, der Beschreibung und den Zeichnungen hervorgehenden Merkmale und Vorteile können sowohl für sich als auch in den verschiedensten Kombinationen erfindungswesentlich sein.

## Patentansprüche

1. Schäumendes Desinfektionsmittel, umfassend:
- mindestens einen einwertigen Alkohol mit 2 bis 4 Kohlenstoffatomen,
- mindestens ein Silikontensid,
- mindestens drei Schaumstabilisatoren ausgewählt aus der Gruppe bestehend aus
i. Citronensäure-Trialkylester, wobei der Alkylrest ein linearer Alkyrest mit 2 bis 5 Kohlenstoffatomen ist,
ii. Alkylethercarboxylat,
iii. einem Gemisch von Neopentylglycol-Diheptanoat und Isododecan,
iv. Triheptanoin,
- optional weitere Hilfsstoffe und
- Wasser.

2. Desinfektionsmittel gemäß Patentanspruch 1, **dadurch gekennzeichnet, dass** die mindestens drei Schaumstabilisatoren ausgewählt sind aus der Gruppe bestehend aus
i. Citronensäure-Trialkylester, bevorzugt Triethylcitrat oder Tributylcitrat,
ii. Kaliumlaurylether-4-Carboxylat oder Natriumlaurylether-5-Carboxylat,
iii. einem Gemisch von Neopentylglycol-Diheptanoat und Isododecan, bevorzugt einem Gemisch von 28 bis 32 Gew.-% Neopentylglycol-Diheptanoat und 68 bis 72 Gew.-% Isododecan und
iv. Triheptanoin.

3. Desinfektionsmittel gemäß Patentanspruch 2, **dadurch gekennzeichnet, dass** die Schaumstabilisatoren
i. Triethylcitrat oder Tributylcitrat und
ii. Kaliumlaurylether-4-Carboxylat oder Natriumlaurylether-5 Carboxylat und mindestens ein dritter Schaumstabilisator aus den Gruppen iii. und iv. sind.

4. Desinfektionsmittel gemäß Patentanspruch 2 oder 3, **dadurch gekennzeichnet, dass** das Desinfektionsmittel die Schaumstabilisatoren in Mengen von
i. 0,1 bis 1 Gew.-% Citronensäure-Trialkylester,
ii. 0,1 bis 1 Gew.-% Alkalimetalllaurylethercarboxylat,
iii. 0,1 bis 1 Gew.-% Gemisch von Neopentylglycol-Diheptanoat und Isododecan, und/oder
iv. 0,1 bis 1 Gew.-% Triheptanoin
enthält, jeweils bezogen auf das Gesamtgewicht des ungeschäumten Desinfektionsmittels.

5. Desinfektionsmittel gemäß einem der vorhergehenden Patentansprüche, **dadurch gekennzeichnet, dass** der einwertige Alkohol Ethanol, 1-Propanol, 2-Propanol oder eine Mischung hiervon ist.

6. Desinfektionsmittel gemäß einem der vorhergehenden Patentansprüche, **dadurch gekennzeichnet, dass** das Desinfektionsmittel 75 Gew.-% bis 90 Gew.-%, bevorzugt 75 bis 87 Gew.-%, Alkohol enthält, bezogen auf das Gesamtgewicht des ungeschäumten Desinfektionsmittels.

7. Desinfektionsmittel gemäß einem der vorhergehenden Patentansprüche, **dadurch gekennzeichnet, dass** das Silikontensid ausgewählt ist aus der Gruppe bestehend aus der PEG/PPG-Dimethicone und PEG-Dimethicone.

8. Desinfektionsmittel gemäß Patentanspruch 7, **dadurch gekennzeichnet, dass** das Silikontensid ausgewählt ist aus der Gruppe bestehend aus Bis-PEG/PPG-20/20-Dimethicon, PEG/PPG-20/6 Dimethicon, PEG/PPG-4/12 Dimethicon, PEG/PPG-14/4 Dimethicon, PEG-14 Dimethicon, einer Mischung aus Bis-PEG/PPG-20/5 PEG/PPG-20/5 Dimethicon, Methoxy PEG/PPG-25/4 Dimethicon und Caprylic/Capric Triglyceriden, PEG-17 Dimethicon, PEG/PPG-18/18 Dimethicon, Bis-PEG-18 Methyletherdimethylsilan, PEG/PPG-25/25 Dimethicon, Cetyl PEG/PPG-10/1 Dimethicon, bevorzugt Bis-PEG/PPG-20/20-Dimethicon.

9. Desinfektionsmittel gemäß einem der vorhergehenden Patentansprüche, **dadurch gekennzeichnet, dass** das Desinfektionsmittel 0,1 Gew.-% bis 5 Gew.-%, bevorzugt 0,5 bis 2 Gew.-%, Silikontensid enthält, bezogen auf das Gesamtgewicht des ungeschäumten Desinfektionsmittels.

10. Desinfektionsmittel gemäß einem der vorhergehenden Patentansprüche, **dadurch gekennzeichnet, dass** das Desinfektionsmittel enthält
75 bis 90 Gew.-% einwertigen Alkohol,
0,5 bis 2 Gew.-% mindestens eines Silikontensids,
1,0 bis 2,0 Gew.-% Schaumstabilisatoren,
0 bis 3 Gew.-%, bevorzugt 0,15 bis 2,5 Gew.-%, weitere Hilfsstoffe und
Wasser,
jeweils bezogen auf das Gesamtgewicht des ungeschäumten Desinfektionsmittels.

11. Desinfektionsmittel gemäß einem der vorhergehenden Patentansprüche, **dadurch gekennzeichnet, dass** das Desinfektionsmittel besteht aus
75 bis 90 Gew.-% einwertigen Alkohol,
0,5 bis 2 Gew.-% mindestens eines Silikontensids,
1,0 bis 2,0 Gew.-% Schaumstabilisatoren,
0,1 bis 1,0 Gew.-% Glycerin oder Glycerinmischung,
0 bis 2 Gew.-%, bevorzugt 0,1 bis 2 Gew.-%, weitere Hilfsstoffe und
Wasser,
jeweils bezogen auf das Gesamtgewicht des ungeschäumten Desinfektionsmittels, wobei sich die Komponenten zu 100 Gew.-% ergänzen.

12. Verwendung eines Desinfektionsmittels gemäß einem der vorhergehenden Patentansprüche zur Händedesinfektion.

13. Desinfektionsmittel gemäß einem der Patentansprüche 1 bis 11 zur Verwendung bei der Händedesinfektion.
